# EUROPÄISCHE PATENTANMELDUNG

(11) **EP 0 629 410 A1**
(43) Veröffentlichungstag der Anmeldung: **21.12.1994**
(21) Anmeldenummer: 93810429.6
(22) Anmeldetag: 15.06.1993
(51) Int. Cl.: A61L 2/24, A61L 2/06

(54) **Sterilisiergerät**

(71) Anmelder: SINTRA HOLDING AG, CH-6210 Sursee (CH)
(72) Erfinder: Lüssi, André, CH-3084 Wabern (CH)
(74) Vertreter: Fischer, Franz Joseph

(57) **Zusammenfassung**

Das Sterilisiergerät umfasst eine Sterilisierkammer (2) zum Aufnehmen von zu sterilisierenden Gegenständen, die bei medizinischen Behandlungen verwendet werden. Die Sterilisierkammer (2) ist mit einer Türe (1) verriegelbar. Eine aufgesetzte Steuereinheit (6) umfasst auf der Frontseite ein Bedienerfeld (9) mit Bedienungselementen (10) und einem Kartenleser (11). Oberhalb des Bedienerfeldes ist ein Anzeigefeld (12) insbesondere zum Anzeigen von Gerätedaten und zum Ausgeben einer Bedienerführung mittels einem LCD-Display angeordnet. Mit Chipkarten, die einen elektronischen Bauteil mit einem nichtflüchtigen Speichermittel aufweisen und die mit dem Kartenleser in eine einen Datenaustausch mit der Steuereinheit erlaubende elektrische Verbindung bringbar sind, können spezielle Sterilisierprogramme gefahren werden, die Bedienerberechtigung des Sterilisiergerätes einfach überwacht sowie weitere Funktionen wie Sperren oder Freigeben von einzelnen Bedienungselementen, einfach vorgenommen werden, ohne dass darunter die Bedienerfreundlichkeit des Gerätes leidet.

## Beschreibung

Die vorliegende Erfindung bezieht sich auf ein Sterilisiergerät gemäss dem Oberbegriff des Patentanspruches 1.

Alle vermehrungsfähigen Mikroorganismen, einschliesslich ihrer zum Teil besonders resistenten Sporen, werden durch Sterilisieren abgetötet. Dies gilt auch Mikroorganismen, die zwar keine Krankheitserreger sind, aber die geschwächte Patienten, z.B. durch pyrogene, d.h. fiebererregende Stoffwechselprodukte, gefährden könnten. Viren werden irreversibel inaktiviert.

Sterilisiert werden darum Gegenstände, die bei medizinischer Behandlung angewandt werden, wenn der natürliche Schutz des Patienten gegen Mikroorganismen beeinträchtigt ist. Dies zum Beispiel an offenen Wunden (bei Operationen nach Unfällen, usw.), in Körperhöhlen und Schleimhäuten (bei der Endoskopie), an ungeschützten Gewebeschichten (nach Verbrennungen) sowie bei veränderter Immunreaktion (nach Transplantationen).

Sterilisiergeräte sind inbesondere dazu geschaffen worden, um Gegenstände zu sterilisieren, die vorher mittels einem speziellen Papier oder mittels einem speziellen Kunststoff eingepackt worden sind.

Bei der häufig angewandten Dampfsterilisation kondensiert Sattdampf, der das Verpackungsmaterial durchdringt, an den zu sterilisierenden Gegenständen. Die Kondensationswärme heizt diese auf. Heisses Kondensat dringt durch die Zellmembranen der Mikroorganismen und greift die Zellkerne direkt an. Sattdampf sterilisiert sehr sicher, trotz verhältnismässig niedriger Sterilisiertemperatur und kurzer Einwirkezeit. Wasserdampf hinterlässt keine giftigen Rückstände an den sterilen Gegenständen.

Die Dampfsterilisation wird bevorzugt angewandt bei Textilien aus Baumwolle oder hitzebeständigen Mischgeweben, bei metallischen Gegenständen mit ausreichendem Korosionsschutz und bei vielen Gegenständen aus Gummi, Kunststoff, Keramik oder Glas. Die Sterilisiertemperatur in diesen Fällen ist üblicherweise 134 Grad. Bei verminderter Temperatur von 120 Grad werden Zellstoff, Flüssigkeiten, empfindliche Gummiwaren, Kunststoffteile, auch Glaswaren und empfindliche mechanische Geräte sterilisiert.

Bei den im Sattdampf zu sterilisierenden Gegenständen ist darauf zu achten, dass diese gegen die auftretenden Temperaturen gegen Feuchtigkeit und gegen die unvermeidlichen Druck- und Temperaturwechsel beständig sind. Organische Materialien sind nicht geeignet, um im Wasserdampf sterilisiert zu werden.

Bei der Kondensation von Sattdampf werden Luft und andere nicht kondensierbare Gase am oder im zu sterilisierenden Gegenstand abgeschieden. Die Entfernung dieser Gase vor der Sterilisation und die Trocknung des sterilisierten Gegenstandes nach der Sterilisation sind die wichtigsten Aufgaben der Technik bei der Dampfsterilisation.

Der zu sterilisierende Gegenstand spielt für den Sterilisiervorgang eine wichtige Rolle. So ist der Sterilisiervorgang abhängig von der Wärmeaufnahme dieses Gegenstandes, also von seiner Temperaturerhöhung, seinem Gewicht und seiner spezifischen Wärme sowie von den Strömungswiderständen, die ein freies Abströmen der Luft behindern. Instrumente und Sondergüter sind schwieriger zu sterilisieren als Textilien. Mit der Zeit haben sich deshalb für verschiedene Arten oder Gattungen von zu sterilisierenden Gegenständen verschiedene Sterilisiervorgänge als geeignet herausgestellt, welche dann in einem für die entsprechende Gattung vorzugsweise anzuwendenden Sterilisierprogramm zusammengefasst worden sind.

Ein Sterilisiergerät umfasst im allgemeinen eine mit einer Tür verschliessbare Sterilisierkammer, welche, Wärme isolierend, vorzugsweise in einer Doppelmantelausführung erstellt ist. Wasserdampfstösse sind unter Druck in die Sterilisierkammer einführbar, wodurch sich darin ein heissfeuchtes Klima mit einer bestimmten Temperatur und mit einem bestimmten Überdruck aufbauen lässt. Mittels einer Vakuumpumpe ist der Druck in der Sterilisierkammer für gewisse Sterilisierphasen ebenfalls absenkbar. In der Sterilisierkammer ist mindestens ein Thermometer und ein Manometer angebracht, um den Temperatur- und den Absolutdruck zu messen. Eine Steuereinheit dient dazu, die einzelnen Schritte oder Sterilisierphasen während einem Programmablauf zu steuern und zu überwachen. Mittels Bedienungselementen können einzelne Sterilisationsprogramme für bestimmte zu sterilisierende Gegenstände ausgewählt und in Gang gesetzt werden. Auf einem Anzeigefeld werden wichtige Daten wie Sterilisierkammertemperatur und Sterilisierkammerdruck angezeigt. Das Sterilisiergerät ist üblicherweise von einer Verschalung, vorzugsweise aus Chromnickelstahl umgeben.

Ein Sterilisiervorgang läuft so ab, dass je nach den zu sterilisierenden Gegenständen vor der eigentlichen Sterilisation, bei der sich in der Sterilisationskammer heisser Wasserdampf unter einem Druck von etwa 2 bis 4 Bar und einer Temperatur von 120 bis etwa 140 Grad während einer Zeit in der Grössenordnung von 1 bis 30 Minuten befindet, mindestens für kurze Zeit wenigstens einmal ein Vakuum aufgebaut wird. Es kann ein fraktioniertes Vakuumverfahren angewandt werden, in welchem nach dem Aufbau des Vakuums durch Einführen von Dampfstössen das erstere wieder abgebaut und anschliessend wieder aufgebaut wird. Dieser Aufbau-Abbau-Zyklus kann einige Male, vorzugsweise etwa dreimal erfolgen. Dadurch wird erreicht, dass Luftblasen, welche oftmals an unzugänglichen Stellen bei den zu sterilisierenden Gegenständen noch vorhanden sein können, mit Sicherheit vor der eigentlichen Sterilisation entfernt werden. Anschliessend an die Sterilisation wird in der Sterilisierkammer wieder ein Vakuum aufgebaut und einige Minuten aufrecht erhalten. Dieses Vakuum dient dazu, die sterilen Gegenstände zu trocknen. Der Trocknungsvorgang ist ebenfalls mit einem fraktionierten Vakuumverfahren möglich.

Es kann für Patienten lebenswichtig sein, dass nur einwandfrei sterile Gegenstände verwendet werden. Die Bedienung des Sterilisationsgerätes bedarf deshalb höchster Aufmerksamkeit und ist nicht von jedermann vorzunehmen. Um dies zu verhindern, um einwandfreie Kontrollen über das mit der Bedienung von Sterilisiergeräten beauftragte Personal zu haben und um Sterilisierprogramme flexibler zu gestalten, hat man nach Verbesserungsmöglichkeiten an Sterilisiergeräten gesucht.

Ein im obigen Sinne verbessertes Sterilisiergerät ist gemäss den Merkmalen im kennzeichnenden Teil des Patentanspruches 1 ausgeführt.

Um die Möglichkeiten der Ein- bzw. Ausgabe von Befehlen bzw. Signalen an die bzw. von der Steuereinheit des Sterilisiergerätes zu erhöhen, hat man die letztere mit einer Daten-Schreib-Lese-Vorrichtung, vorzugsweise mit einem Kartenleser, ausgestattet. Mittels Datenträgerorganen oder sogenannten Chipkarten, auf welchen Informationen speicherbar und wieder abrufbar sind, und von welchen eine unbegrenzte Anzahl vorhanden sein kann, können am Sterilisiergerät eine Vielzahl von zusätzlichen Funktionen und/oder Überwachungsaufgaben vorgenommen werden, ohne dass dazu die Einfachheit der Bedienung beeinträchtigt wird, wie dies beispielsweise dann der Fall wäre, wenn anstelle des Kartenlesers mit zusätzlichen Bedienungselementen, die im Bedienungsfeld angeordnet wären, operiert werden müsste. Trotz den vielen Mehrfunktionen, die mit den Chipkarten und dem Kartenleser realisierbar sind, wird die Bedienung des Sterilisiergerätes nicht erschwert sondern eher erleichtert.

Die Chipkarten können in mehrere Kategorien aufgeteilt sein, wobei je nach Kategorie unterschiedliche Daten in deren Speichermittel abgespeichert sind. Es können beispielsweise sogenannte Bedienpersonalkarten und Chefkarten unterschieden werden. Im zweiten Fall kann anhand eines entsprechenden Identifikationscodes, der im Speichermittel der entsprechenden Chefkarte gespeichert ist und von der Steuereinheit des Sterilisiergerätes erkannt wird, bewirkt werden, dass der Inhaber dieser Chefkarte einzelne Funktionen am Sterilisiergerät ändern kann, wie beispielsweise einzelne Bedienungselemente, die zum Auswählen von Sterilisationsprogrammen bestimmt sind, Sperren oder Freigeben oder festlegen, dass nur Bedienpersonal mit Chipkarten, die ganz bestimmte Bedieneridentifikationen aufweisen, zum Bedienen des Sterilisationsgerätes berechtigt sind. Es kann im weiteren mit einer Chefkarte jederzeit festgestellt werden, welche Bediener zum Bedienen des entsprechenden Sterilisiergerätes berechtigt sind. Für die berechtigten Bediener kann der zugeordnete Identifikationscode oder eine Berechtigungsnummer beispielsweise auf einem LCD-Display, welches in einem Anzeigefeld vorhanden ist, angezeigt werden. Diese Informationen können ebenfalls an einen an das Sterilisiergerät anschliessbaren Drucker oder von einem im Sterilisiergerät eingebauten Drucker ausgegeben werden.

Mit den Chipkarten des ersten Falles, die für das Bedienpersonal vorgesehen sind, kann erreicht werden, dass die Bedienungselemente für das Sterilisiergerät erst aktiv werden können, wenn nach dem Einführen der der entsprechenden Person zugeordneten Chipkarte deren Berechtigung anhand eines Identifikationscodes, der sowohl im Speichermittel auf der Chipkarte als auch im Speichermittel der Steuereinheit vorhanden ist, festgestellt worden ist. Es kann dadurch verhindert werden, dass unberechtigte Personen unsachgemässe Sterilisationsvorgänge durchführen.

Es ist ebenfalls möglich, dass spezielle Sterilisationsprogramme auf einer der Chipkarten enthalten sind, wobei die Steuereinheit nach dem Einführen einer solchen Chipkarte in den Kartenleser anhand eines anderen Identifikationscodes erkennt, dass auf der Chipkarte ein spezielles Sterilisierprogramm gespeichert ist, das jetzt ausgeführt werden soll. Die Programmdaten werden in die Steuereinheit eingelesen und die einzelnen Sterilisierphasen gemäss diesem speziellen Programm abgearbeitet.

Die im Speichermittel der Steuereinheit abgespeicherte Konfiguration bzw. die darin festgelegten Initialdaten für das Sterilisiergerät können mit sogenannten Datensicherungschipkarten in deren Speichermittel übertragen werden. Nach einem eventuellen Ausfall des entsprechenden Sterilisiergerätes braucht dieses nicht wieder in mühsamer Kleinarbeit neu konfiguriert bzw. initialisiert zu werden, sondern es können mit der Datensicherungschipkarte sämtliche Daten, die vorher im Speichermittel der Steuereinheit gespeichert waren, wieder diesem Speichermittel zugeführt werden. Zu diesen Daten können ebenfalls die verschiedenen Berechtigungscodes und/oder Identifikationscodes gehören.

Anhand eines Ausführungsbeispieles ist die vorliegende Erfindung im folgenden näher beschrieben. Es zeigen
**Fig. 1** eine Aussenansicht eines Tischsterilisiergerätes,
**Fig. 2** das Bedienerfeld und das Anzeigefeld des Sterilisiergerätes gemäss der Fig. 1 vergrössert in einer Frontansicht,
**Fig. 3** einen Kartenleser mit einer Chipkarte, und
**Fig. 4** ein Blockschaltbild eines Teiles der Steuereinheit.

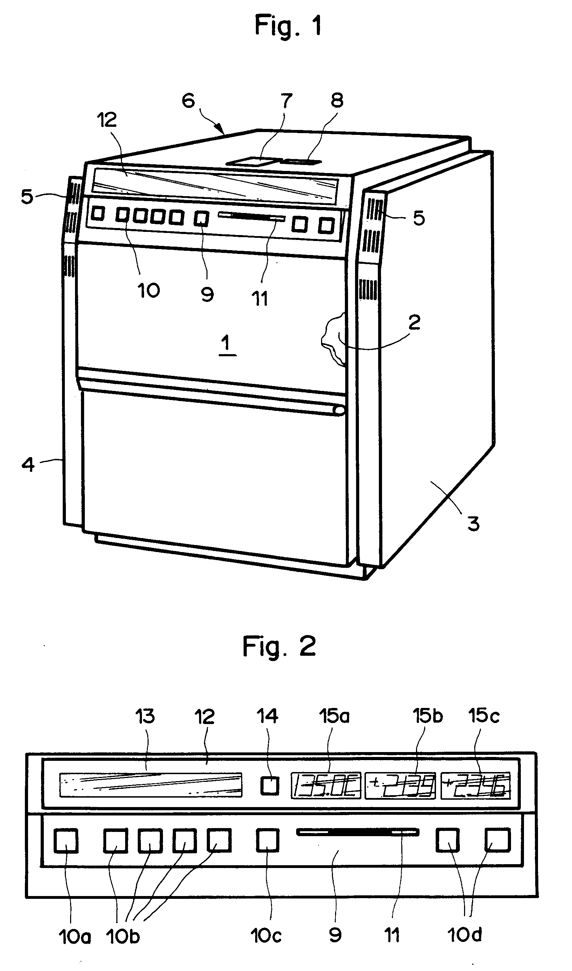

Das in der Fig. 1 als Beispiel gezeigte Sterilisiergerät ist ein Tischsterilisator, der nach dem gleichen Sterilisierverfahren arbeitet wie Grossanlagen. Das Gerät ist vorgesehen, für den Einsatz in Arzt- und Zahnarztpraxen, Spitälern und allgemein allen Bereichen, wo chirurgische Eingriffe vorgenommen werden.

Das Sterilisiergerät umfasst eine handbetätigbare Türe 1, die als vertikal nach unten öffnende Schiebetüre ausgeführt ist. Mit der Tür kann eine Sterilisierkammer 2 geöffnet werden, in welche die zu sterilisierenden Gegenstände eingeführt werden können. Die Türe ist mit einer Sicherheitsverriegelung ausgestattet, welche es verunmöglicht, das Sterilisiergerät in Gang zu setzen, bevor die Türe geschlossen und verriegelt ist oder die Türe zu öffnen, bevor ein laufendes Sterilisierprogramm abgearbeitet worden ist. Die Sterilisierkammer 2 selbst ist aus Wärmeisolationsgründen von einem Doppelmantel umgeben. Das ganze Gerät ist in eine Verschalung aus rostfreiem Stahl eingebaut. In den beiden Seitenwänden 3 und 4 sind mit 5 Kühlöffnungen gezeigt, die zum Aufrechterhalten einer Luftkonvektionskühlung bestimmt sind. Oben am Sterilisiergerät ist eine Steuereinheit 6 aufgesetzt. Ein eingebauter Ventilator 7 dient zusammen mit den Kühlöffnungen 5 für die Kühlung der in der Steuereinheit eingebauten Bauelemente auf eine optimale Betriebstemperatur.

Mit 8 ist eine Druckstreifenausgabe gekennzeichnet, aus welcher von einem eingebauten Nadeldrucker bedrucktes Papier ausgebbar ist. Die einzelnen Sterilisierphasen jedes Sterilisierprogrammes werden nach dem Programmende ausgedruckt. Das Druckprotokoll wird abgeschnitten und durch die Druckstreifenausgabe ausgestossen. Fehlermeldungen werden sofort nach dem Auftreten in roter Schrift gedruckt. Sie sind nach Programmende auf dem Druckprotokoll ersichtlich.

Auf der Frontseite der Steuereinheit 6 ist ein Bedienerfeld 9 angeordnet, welches mehrere Bedienungselemente 10, auf die später genauer eingegangen wird, sowie eine Daten-Schreib-Lese-Vorrichtung, ein Kartenleser 11, umfasst. Mit 12 ist ein Anzeigefeld gekennzeichnet, welches oberhalb dem Bedienerfeld angeordnet ist.

Das Bedienerfeld 9 und das Anzeigefeld 12 sind in der Fig. 2 in einer Frontansicht vergrössert dargestellt. So sind im Bedienerfeld 9 von links nach rechts eine Ein-/Austaste 10a, vier Programmwahltasten 10b, eine Shifttaste 10c, der bereits erwähnte Kartenleser 11 und zwei Steuertasten 10d angeordnet. Im Anzeigefeld 12 ist links ein LCD-Display 13, insbesondere zur Benutzerführung und zur Anzeige der Sterilisierprogrammphasen im Klartext vorhanden. Mit 14 ist eine Kontrolleuchte bezeichnet, die solange aufleuchtet, wie in der Sterilisierkammer ein Überdruck herrscht. Mit 15a - 15c sind weitere Anzeigen bezeichnet, die aus Siebensegmentelementen aufgebaut sind. Bei 15a wird die Sterilisierkammertemperatur in Grad Celsius angezeigt, bei 15b der Sterilisierkammerdruck in Bar und bei 15c der Druck im Dampferzeuger, ebenfalls in Bar.

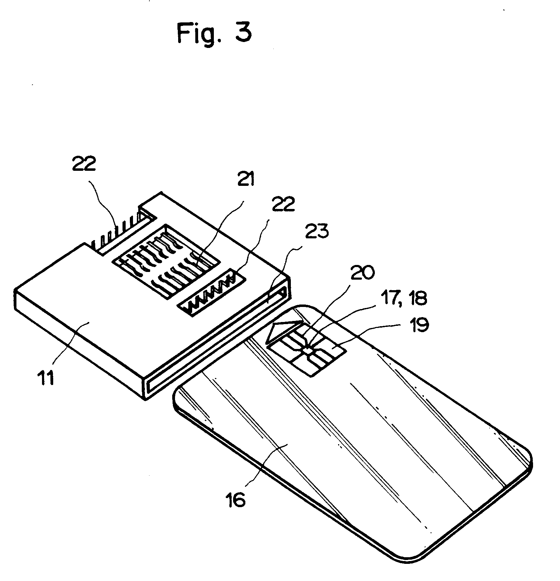

In der Fig. 3 sind der Kartenleser 11 und eine Chipkarte 16 dargestellt. Eine Einführungsöffnung 23 des Kartenlesers 11 ist der Chipkarte 16 zugewandt, die neben dem Kartenleser gelegen, gezeigt ist. Auf der Chipkarte 16 ist ein Elektronikbauteil 17 angeordnet, welcher mindestens ein Speichermittel 18 umfasst. Üblicherweise ist das Speichermittel als EEPROM ausgeführt. Dies ist ein nichtflüchtiger Halbleiterspeicher, der elektrisch schreibbar und löschbar ist. Im Kartenleser 11 sind im wesentlichen zwei Reihen Abtastkontakte 21 enthalten, welche zu Anschlussfahnen 22, die ebenfalls in zwei Reihen angeordnet sind, führen. Von den Anschlussfahnen wird der Kartenleser über ein Verbindungskabel mit einer Steuerelektronik verbunden.

Der Elektronikbauteil 17 auf der Chipkarte 16 ist mit einer metallisierten Kontaktfläche 19 abgedeckt. Diese ist durch einzelne Isolationsbahnen 20 derart in Teilflächen aufgeteilt, dass bei in die Einführungsöffnung 23 des Kartenlesers 11 eingeführter Chipkarte 16 je ein Abtastkontakt 21 je einer der Teilflächen zugeordnet ist. Ein Abtastvorgang wird erst freigegeben, wenn ein im Kartenleser vorhandener Endlagenkontakt, der in den Figuren nicht sichtbar ist, das vollständige Einschieben der Chipkarte meldet. Jeder der metallisierten Teilflächen ist mit einem Anschluss des auf der Chipkarte 16 angeordneten Elektronikbauteiles 17 verbunden.

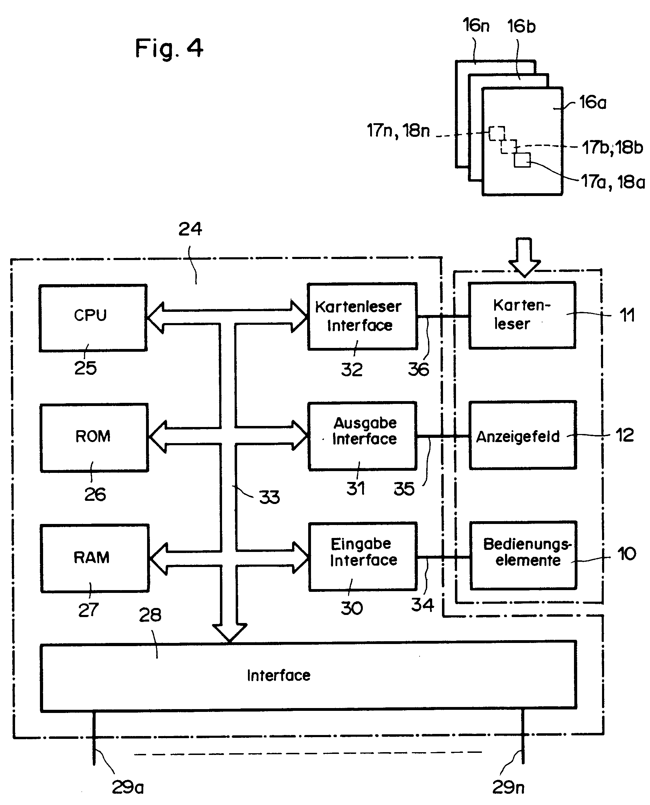

In der Fig. 4 ist mit 24 ein Teil der Steuereinheit 6 bezeichnet. Es ist derjenige Teil der Steuereinheit mit dem Rechenmittel 75, den Speichermittel 26, 27 und den Interfacestromkreisen 28, 30, 31 und 32, welche mit den anderen elektrischen Teilen der Steuereinheit elektrisch verbunden sind. Die Speichermittel 26 und 27 sind in einen ROM-Speicher 26, in welchem der Programmcode zum Betrieb des Rechenmittels gespeichert ist und in einen RAM-Speicher 27 aufgeteilt. Im letzteren ist mindestens ein nichtflüchtiger batteriegepufferter Bereich vorhanden, in welchem Konfigurationsdaten und Initialdaten sowie Identifikationscodes und Berechtigungsnummern gespeichert sind. Das Interface 28 enthält Stromkreise, die über elektrische Leitungen 29a bis 29n mit anderen Steuerelementen der Steuereinheit verbunden sind. So werden von diesem Interface Signale abgegeben zum Steuern der Vakuumpumpe, des Dampferzeugers, zum Verriegeln der Türe, etc. und es werden Kontrollsignale von den in der Sterilisierkammer eingebauten Messfühlern sowie anderen Überwachungssensoren empfangen und dem Rechenmittel in einem geeigneten Format zur Weiterverarbeitung zugeleitet. Die auf dem Bedienerfeld 9 angeordneten Bedienungselemente sind über ein erstes elektrisches Verbindungskabel 34 mit dem Eingabeinterface 30 verbunden. An das Ausgabeinterface 31 ist über ein zweites elektrisches Verbindungskabel 35 das Anzeigefeld 12 mit seinen verschiedenen Anzeigen angeschlossen. Der Kartenleser 11 steht über ein drittes elektrisches Verbindungskabel 36 mit dem Kartenleserinterface 32 in Verbindung. Mit 16a - 16n sind eine Vielzahl von Chipkarten dargestellt, welche je in ihrem weiteren Speichermittel 18a - 18n des Elektronikbauteiles 17a - 17n Daten zum Steuern, Konfigurieren und/oder Bedienen des Sterilisiergerätes enthalten können.

Mit 33 sind Busverbindungen gezeigt, mit welchen die vorgenannten Bausteine 25, 26, 27, 28, 30, 31 und 32 des mit 24 bezeichneten Teiles der Steuereinheit miteinander verbunden sind. Wie in der Mikroprozessortechnik üblich, sind die Busverbindungen in einen aus der Figur nicht sichtbaren Adressen-, Daten- und Steuerbus aufgeteilt.

Die eingangs beschriebenen Funktionen der einzelnen Chipkarten, insbesondere zum Sperren oder Freigeben von einzelnen Bedienungselementen, zum Sperren oder Freigeben des Druckers oder zum Eingeben von Identifikationscodes und Berechtigungsnummern, werden nach dem Einführen einer entsprechenden Chipkarte in den Kartenleser 11 mit den Steuertasten 10d und der Shifttaste 11c durchgeführt.

Selbstverständlich ist es möglich, anstelle des hier beispielsweise beschriebenen Kartenlesers und der Chipkarten andere Vorrichtungen zum Ein- und Auslesen von Daten zu verwenden, wenn diese einfach in der Handhabung und preisgünstig sind.

## Patentansprüche

1. Sterilisiergerät, insbesondere für Gegenstände, die bei medizinischen Behandlungen verwendet werden, mit einer Sterilisierkammer (2), die mit einer Tür (1) verschliessbar ist, einer Steuereinheit (6) zum Steuern und Ueberwachen verschiedener Sterilisierprogramme, mit Bedienungselementen (10a - 10d) zum Eingeben von Steuersignalen an die Steuereinheit, sowie mit einem Anzeigefeld (12) zum Ausgeben einer Bedienerführung und zum Anzeigen von Zuständen, dadurch gekennzeichnet, dass die Steuereinheit (6) ein Rechenmittel (25) und Speichermittel (26, 27) aufweist, dass eine Daten-Schreib-Lese-Vorrichtung (11) und mehrere Datenträgerorgane (16a - 16n) vorhanden sind, wobei jedes Datenträgerorgan mit einem nichtfflüchtigen weiteren Speichermittel (18a - 18n) ausgerüstet und mit der Daten-Schreib-Lese-Vorrichtung (11) in eine einen Datenaustausch erlaubende Verbindung bringbar ist.

2. Gerät nach Anspruch 1, dadurch gekennzeichnet, dass die Daten-Schreib-Lese-Vorrichtung ein Kartenleser (11) ist und die Datenträgerorgane in den Kartenleser einführbare und mit dem letzteren elektrisch verbindbare Chipkarten (16a - 16n) sind, dass der Kartenleser (11) mit der Steuereinheit (6) elektrisch verbunden ist, wobei zwischen der Steuereinheit (6) und einer in den Kartenleser (11) eingeführten Chipkarte (16a - 16n) Daten austauschbar sind.

3. Gerät nach Anspruch 1 oder 2, dadurch gekennzeichnet, dass die Datenträgerorgane (16a - 16n) in mehrere Kategorien aufgeteilt sind, wobei je nach Kategorie unterschiedliche Daten im weiteren Speichermittel (18a - 18n) gespeichert sind.

4. Gerät nach einem der Ansprüche 1 bis 3, dadurch gekennzeichnet, dass ein erster Teil der Datenträgerorgane (16a - 16n) für das Bedienpersonal des Gerätes bestimmt ist, wobei im weiteren Speichermittel (18a - 18n) dieser Datenträgerorgane ein erster Identifikationscode derart enthalten ist, dass erst nach dem Lesen dieses Codes mit der Daten-Schreib-Lese-Vorrichtung (11) und dessen Ueberprüfung in der Steuereinheit (6) die Bedienungselemente (10a - 10d) zum Eingeben von Steuersignalen bereit sind.

5. Gerät nach einem der Ansprüche 1 bis 4, dadurch gekennzeichnet, dass ein zweiter Teil der Datenträgerorgane (16a - 16n) für das Chefpersonal bestimmt ist, wobei im weiteren Speichermittel (18a - 18n) dieser Datenträgerorgane ein zweiter Identifikationscode derart enthalten ist, dass nach dem Lesen dieses Codes mit der Daten-Schreib-Lese-Vorrichtung (11) und dessen Ueberprüfung in der Steuereinheit (6) einzelne Bedienungselemente (10a - 10d) für die weitere Benutzung sperrbar oder freigebbar sind, Daten in die Steuereinheit (6) eingebbar sind, anhand welchen festlegbar ist, wer für die Bedienung des Gerätes berechtigt ist und/oder die Berechtigungsdaten auf dem Anzeigefeld (12) oder einem an das Gerät anschliessbaren oder im Gerät vorhandenen Drucker ausgebbar sind.

6. Gerät nach einem der Ansprüche 1 bis 5, dadurch gekennzeichnet, dass ein dritter Teil der Datenträgerorgane (16a - 16n) dazu bestimmt ist, im weiteren Speichermittel (18a - 18n) dieser Datenträgerorgane gespeicherte spezielle Sterilisierprogramme über die Daten-Schreib-Lese-Vorrichtung (11) an die Steuereinheit (6) zu übergeben.

7. Gerät nach einem der Ansprüche 1 bis 6, dadurch gekennzeichnet, dass ein vierter Teil der Datenträgerorgane (16a - 16n) dazu bestimmt ist, im weiteren Speichermittel (18a - 18n) die im Speichermittel (27) der Steuereinheit (6) gespeicherten Daten der Gerätekonfiguration abzuspeichern oder im weiteren Speichermittel (18a - 18n) abgespeicherte Daten für eine Gerätekonfiguration an das Speichermittel (27) in der Steuereinheit (6) zu übergeben.
